# EUROPEAN PATENT APPLICATION

(11) **EP 3 636 267 A1**
(43) Date of publication of application: **15.04.2020**
(21) Application number: 18814378.8
(22) Date of filing: 05.06.2018
(51) Int. Cl.: A61K 31/7034, A61K 8/60, A61K 8/73, A61K 31/716, A61P 17/02, A61P 17/16, A61P 19/02, A61P 43/00, A61Q 19/00

(54) **GLYCOSAMINOGLYCAN-PRODUCING PROMOTER AND COMPOSITION FOR PROMOTING GLYCOSAMINOGLYCAN PRODUCTION**

(30) Priority: 05.06.2017 JP 2017110803
(71) Applicant: Showa Denko K.K., Tokyo 105-8518 (JP)
(72) Inventor: NAKAGAMI Yuko, Tokyo 105-8518 (JP); MISHINA Natsuno, Tokyo 105-8518 (JP); KATO Eiko, Tokyo 105-8518 (JP)
(74) Representative: Strehl Schübel-Hopf & Partner
(86) International application number: PCT/JP2018/021551
(87) International publication number: WO 2018/225728

(57) **Abstract**

A glycosaminoglycan-producing promoter contains, as an active ingredient, an inositol derivative in which a sugar binds to inositol. In addition, a composition for promoting glycosaminoglycan production contains the above-mentioned glycosaminoglycan-producing promoter, and a pharmaceutically acceptable carrier.

## Description

### [Technical Field]

The present invention relates to a glycosaminoglycan-producing promoter, and a composition for promoting glycosaminoglycan production.

Priority is claimed on Japanese Patent Application No. 2017-110803, filed June 5, 2017, the content of which is incorporated herein by reference.

### [Background Art]

Glycosaminoglycans are acidic mucopolysaccharides consisting of repeating disaccharide structures in which a uronic acid or galactose binds to an amino sugar. Glycosaminoglycans are classified according to the types of saccharides they contain, and examples thereof include hyaluronic acid, keratan sulfate, heparan sulfate, heparin, chondroitin, chondroitin sulfate, dermatan sulfate, and the like. They are present in various sites in a living body as a matrix substance (extracellular matrix) between fibers and cells of tissue.

A molecule in which a glycosaminoglycan covalently binds to core proteins as a sugar chain is a glycoprotein called a proteoglycan, which binds a large amount of water, and thereby protects the fiber components of tissue with its elasticity, forms cartilage, or binds to adhesion molecules. In addition, glycosaminoglycans are known to bind to proteins and lipids as a sugar chain part of proteoglycans to form glycoproteins and glycolipids, and play an important role in the stabilization of proteins and lipids to which they bind and information transmission between cells as a protein label.

Currently, as agents promoting production of glycosaminoglycans, the following are known: a milk basic protein (Patent Literature 1), peptides or derivatives thereof or salts thereof (Patent Literature 2), a mixture of Madonna Lily extract and glucosamine (Patent Literature 3), lysophospholipids (Non-Patent Literature 1), *Aloe arborescens* extract (Non-Patent Literature 2), and polystyrene derivatives having sugar side chains (Non-Patent Literature 3). However, the effects and practical applications of these agents are still not sufficient because they contribute to promoting production of only hyaluronic acid among glycosaminoglycans.

### [Citation List]

### [Patent Literature]

### [Patent Literature 1]

Japanese Unexamined Patent Application, First Publication No. 2016-124852

### [Patent Literature 2]

PCT International Publication No. WO2014/157485

### [Patent Literature 3]

Japanese Unexamined Patent Application, First Publication No. 2013-203725

### [Non-Patent Literature]

### [Non-Patent Literature 1]

Shinji TANAKA et al., Journal of Japan Oil Chemists' Society, 46 (9), p969-975, 1997

### [Non-Patent Literature 2]

Yoshiko TSURUMI et al., J. Soc. Cosmet. Chem. Japan, 35 (3), p243-248, 2001

### [Non-Patent Literature 3]

Hideo KURODA et al., J. Soc. Cosmet. Chem. Japan, 33 (1), p39-47, 1999

### [Summary of Invention]

### [Technical Problem]

As described above, most of the glycosaminoglycan-producing promoters that have been reported so far are promoters that promote production of only one type of glycosaminoglycan such as hyaluronic acid, and many of them exhibit insufficient effects.

Accordingly, an object of the present invention is to provide a glycosaminoglycan-producing promoter and a composition for promoting glycosaminoglycan production which can promote the production of both a sulfated glycosaminoglycan and hyaluronic acid.

### [Solution to Problem]

The present invention includes the following aspects.
(1) A glycosaminoglycan-producing promoter containing, as an active ingredient, an inositol derivative in which a sugar binds to inositol.
(2) The glycosaminoglycan-producing promoter according to (1), in which the sugar is a monosaccharide or an oligosaccharide.
(3) The glycosaminoglycan-producing promoter according to (2), in which the monosaccharide is glucose.
(4) The glycosaminoglycan-producing promoter according to (2), in which the oligosaccharide is an oligosaccharide containing glucose as a structural unit.
(5) The glycosaminoglycan-producing promoter according to any one of (1) to (4), in which the inositol is myo-inositol.
(6) The glycosaminoglycan-producing promoter according to any one of (1) to (5), in which the glycosaminoglycan is a sulfated glycosaminoglycan.
(7) The glycosaminoglycan-producing promoter according to any one of (1) to (5), in which the glycosaminoglycan is hyaluronic acid.
(8) A composition for promoting glycosaminoglycan production, containing: the glycosaminoglycan-producing promoter according to any one of (1) to (7), and a pharmaceutically acceptable carrier.
(9) The composition for promoting glycosaminoglycan production according to (8), in which the content of the inositol derivative is 0.01% to 50% by mass.
(10) The composition for promoting glycosaminoglycan production according to (8) or (9), which is an external preparation for skin.
(11) The composition for promoting glycosaminoglycan production according to any one of (8) to (10), which is a cosmetic preparation.

### [Advantageous Effects of Invention]

According to the present invention, it is possible to provide a glycosaminoglycan-producing promoter and a composition for promoting glycosaminoglycan production which can promote the production of both a sulfated glycosaminoglycan and hyaluronic acid.

### [Description of Embodiments]

### [Glycosaminoglycan-producing promoter]

In one embodiment, the present invention provides a glycosaminoglycan-producing promoter containing, as an active ingredient, an inositol derivative in which a sugar binds to inositol.

As will be described later in Examples, the glycosaminoglycan-producing promoter of the present embodiment can promote the production of both a sulfated glycosaminoglycan and hyaluronic acid. The glycosaminoglycan-producing promoter of the present embodiment is particularly effective for promoting the production of glycosaminoglycan in fibroblasts. By such action of promoting glycosaminoglycan production, the glycosaminoglycan-producing promoter of the present embodiment can effectively ameliorate diseases or disorders caused due to glycosaminoglycan deficiency. Accordingly, the glycosaminoglycan-producing promoter of the present embodiment can be said to be an agent for ameliorating diseases or disorders caused due to glycosaminoglycan deficiency. In the present specification, the term "diseases or disorders caused due to glycosaminoglycan deficiency" refers to diseases or disorders caused due to glycosaminoglycan deficiency in tissue, such as connective tissue, which occur due to inhibition of glycosaminoglycan production in fibroblasts and the like, degradation of glycosaminoglycans in tissue, and the like. The diseases or disorders caused due to glycosaminoglycan deficiency are not particularly limited, but examples thereof include decreased skin function (such as residual acne scars), pigmentation, osteoarthrosis, pain disorders (such as arthritis, lower back pain, and nerve pain), and the like.

In addition, since the glycosaminoglycan-producing promoter of the present embodiment acts to promote glycosaminoglycan production as described above, it can also be used for applications such as promotion of healing of wounds and burn injuries. Accordingly, the glycosaminoglycan-producing promoter of the present embodiment can be said to be a wound-healing promoter or a burn-injury-healing promoter.

Furthermore, as will be described later in Examples, the glycosaminoglycan-producing promoter of the present embodiment is particularly effective for promoting the production of N-sulfated glycosaminoglycan and hyaluronic acid. Accordingly, the glycosaminoglycan-producing promoter of the present embodiment can be said to be an N-glycosaminoglycan-producing promoter or a hyaluronic-acid-producing promoter.

### (Inositol derivatives)

The glycosaminoglycan-producing promoter of the present embodiment contains, as an active ingredient, inositol derivatives in which a sugar binds to inositol.

Inositol is a cyclic hexahydric alcohol represented by C₆H₆(OH)₆. There are nine stereoisomeric forms of inositol: cis-inositol, epi-inositol, allo-inositol, myo-inositol, muco-inositol, neo-inositol, chiro-inositol (D-chiro- and L-chiro-), and scyllo-inositol.

In the glycosaminoglycan-producing promoter of the present embodiment, the inositol constituting the inositol derivatives is preferably myo-inositol, which is the only one that is physiologically active among the above-mentioned isomeric forms. Inositol can be synthesized by a method of extraction from rice bran, a chemical synthesis method, a fermentation method, or the like.

In the glycosaminoglycan-producing promoter of the present embodiment, the inositol derivatives are compounds in which a sugar binds to a hydroxyl group of inositol. The sugar may bind to any one of six hydroxyl groups present in an inositol molecule, or may bind to any two or more thereof.

A sugar that binds to inositol may be a monosaccharide or an oligosaccharide. For example, one or more monosaccharides may bind to one inositol molecule, one or more oligosaccharides may bind to one inositol molecule, or one or more monosaccharides and one or more oligosaccharides may bind to one inositol molecule. In the inositol derivatives, a total number of monosaccharides or oligosaccharides binding to one inositol molecule is 1 or more, may be 2 or more for example, may be 3 or more for example, and may be 4 or more for example, in terms of monosaccharide units.

In the present specification, a monosaccharide refers to a sugar group that cannot be further hydrolyzed, and refers to a compound that is a constituent element when forming a polysaccharide. A monosaccharide can also be said to be the smallest structural unit of a sugar group. In the present specification, the term "monosaccharide unit" refers to a chemical structure corresponding to a monosaccharide. A "monosaccharide unit" can also be said to be a chemical structure derived from a monosaccharide. For example, a disaccharide is converted into two monosaccharide units, and a trisaccharide is converted into three monosaccharide units. More specifically, for example, mannitol, sorbitol, xylitol, erythritol, pentaerythritol, glucose, fructose, xylose, or the like is converted into one monosaccharide unit.

In addition, maltitol, sucrose, lactose, maltose, trehalose, or the like is converted into two monosaccharide units. Furthermore, for example, α-cyclodextrin is converted into six monosaccharide units; β-cyclodextrin is converted into seven monosaccharide units; and γ-cyclodextrin is converted into eight monosaccharide units.

The inositol derivatives may be a mixture of inositol derivatives to which different numbers of sugars bind in terms of monosaccharide units. For example, the inositol derivatives may be a mixture of an inositol derivative in which one saccharide in terms of monosaccharide units binds to one inositol molecule, an inositol derivative in which two saccharides in terms of monosaccharide units bind to one inositol molecule, an inositol derivative in which three saccharides in terms of monosaccharide units bind to one inositol molecule, an inositol derivative in which four saccharides in terms of monosaccharide units bind to one inositol molecule, and an inositol derivative in which five or more saccharides in terms of monosaccharide units bind to one inositol molecule. For example, the inositol derivatives may be inositol derivatives containing 10% to 100% by mass of inositol derivatives in which two or more saccharides in terms of monosaccharide units bind to one inositol molecule with respect to a total mass (100%) of the inositol derivatives. A proportion of the inositol derivatives in which two or more saccharides in terms of monosaccharide units bind to one inositol molecule in the total mass (100%) of the inositol derivatives is, for example, 20% by mass or more, 30% by mass or more, 40% by mass or more, 50% by mass or more, 60% by mass or more, 70% by mass or more, or 80% by mass or more.

A sugar constituting the inositol derivatives is not particularly limited, and examples thereof include mannitol, sorbitol, xylitol, maltitol, erythritol, pentaerythritol, glucose, sucrose, fructose, lactose, maltose, xylose, trehalose, α-cyclodextrin, β-cyclodextrin, γ-cyclodextrin, and the like.

The sugar constituting the inositol derivatives may be glucose, or an oligosaccharide containing glucose as a structural unit. The oligosaccharide may contain only glucose as a structural unit. Alternatively, the oligosaccharide may contain at least one glucose molecule, and a sugar other than glucose as structural units. A molecular weight of the oligosaccharide may be, for example, about 300 to 3000. More specific examples of oligosaccharides include disaccharides such as sucrose, lactose, maltose, trehalose, and cellobiose; trisaccharides such as raffinose, melezitose, and maltotriose; tetrasaccharides such as stachyose; and the like.

The inositol derivatives may be a mixture of an inositol derivative in which the sugar is a monosaccharide, and an inositol derivative in which the sugar is an oligosaccharide. The inositol derivatives may be a mixture of inositol derivatives to which different types of sugars bind.

From the viewpoint of easily obtaining highly purified inositol derivatives, it is preferable to use β-cyclodextrin which is industrially inexpensive and can be stably supplied as a raw material for the inositol derivatives. In this case, the sugar constituting the inositol derivatives contains glucose as a structural unit. Meanwhile, when cheaper starch or the like is used as a raw material for the inositol derivatives, various sugars are transferred to various places during synthesis of inositol derivatives, and thus a degree of purification of inositol derivatives to be obtained tends to become unstable.

In addition, the inositol derivative may be in a form of a pharmaceutically acceptable salt. In the present specification, the term "pharmaceutically acceptable salt" refers to a salt form that does not inhibit the effect of promoting glycosaminoglycan production by the inositol derivative. The pharmaceutically acceptable salts of the inositol derivatives are not particularly limited, and examples thereof include salts with alkali metals (sodium, potassium, and the like); salts with alkaline earth metals (magnesium, calcium, and the like); salts with organic bases (pyridine, triethylamine, and the like); salts with amines; salts with organic acids (acetic acid, formic acid, propionic acid, fumaric acid, maleic acid, succinic acid, tartaric acid, citric acid, malic acid, oxalic acid, benzoic acid, methanesulfonic acid, and the like); salts with inorganic acids (hydrochloric acid, phosphoric acid, hydrobromic acid, sulfuric acid, nitric acid, and the like); and the like.

In addition, the inositol derivative may be in a form of a solvate. Furthermore, the inositol derivative may be in a form of a solvate of salt of the inositol derivative. The solvate is not particularly limited, but examples thereof include hydrates, ethanol solvates, and the like.

### (Method for synthesizing inositol derivatives)

A method for synthesizing the inositol derivatives is not particularly limited, and the inositol derivatives can be appropriately synthesized by a known method of the related art. For example, the inositol derivatives may be synthesized by reacting inositol with cyclodextrin, which is one kind of oligosaccharides, in the presence of cyclodextrin glucanotransferase (refer to, for example, Japanese Unexamined Patent Application, First Publication No. S63-196596). Alternatively, the inositol derivatives may be synthesized by a method for obtaining glucosyl derivatives using glucosyl phosphite as a sugar donor (refer to, for example, Japanese Unexamined Patent Application, First Publication No. H6-298783).

The glycosaminoglycan-producing promoter of the present embodiment may contain, as the inositol derivatives, one of compounds selected from the group consisting of the above-mentioned inositol derivatives, salts of the inositol derivatives, and solvates thereof, or may contain two or more kinds thereof in combination.

The glycosaminoglycan-producing promoter of the present embodiment can itself be administered to patients to be used for the purpose of ameliorating diseases or disorders caused due to glycosaminoglycan deficiency, such as decreased skin function (such as residual acne scars), pigmentation, osteoarthrosis, and pain disorders (such as arthritis, lower back pain, and nerve pain); or for the purpose of promoting healing of wounds or burn injuries. In addition, the glycosaminoglycan-producing promoter of the present embodiment can be blended into pharmaceuticals, cosmetic preparations, and the like to be used for the purpose of imparting a function of ameliorating diseases or disorders caused due to glycosaminoglycan deficiency, or for the purpose of imparting a function of promoting healing of wounds or burn injuries. Furthermore, it can be blended into a composition for promoting glycosaminoglycan production to be described later and used.

The glycosaminoglycan-producing promoter of the present embodiment may be administered to a patient at high risk of developing a disease or disorder caused due to glycosaminoglycan deficiency, such as decreased skin function (such as residual acne scars), pigmentation, osteoarthrosis, and pain disorders (such as arthritis, lower back pain, and nerve pain), to prevent the diseases or disorders caused due to glycosaminoglycan deficiency. In addition, the glycosaminoglycan-producing promoter of the present embodiment may be administered to a patient, who has developed a disease or disorder caused due to glycosaminoglycan deficiency, to be used for treating the disease or disorder caused due to glycosaminoglycan deficiency. Furthermore, it may also be used for treating a wound or burn injury of a patient with a wound or burn injury.

The glycosaminoglycan-producing promoter of the present embodiment can be administered to a patient in the same manner as the composition for promoting glycosaminoglycan production to be described later, and it is preferably used as an external preparation for the skin.

### [Composition for promoting glycosaminoglycan production]

In one embodiment, the present invention provides a composition for promoting glycosaminoglycan production, containing the above-described glycosaminoglycan-producing promoter, and a pharmaceutically acceptable carrier.

The composition for promoting glycosaminoglycan production of the present embodiment can be manufactured by mixing the above-described glycosaminoglycan-producing promoter, the pharmaceutically acceptable carrier, and optionally, other ingredients, and formulating them according to a general method (for example, a method described in the Japanese Pharmacopoeia).

In the present specification, the term "pharmaceutically acceptable carrier" refers to a carrier that does not inhibit physiological activity of an active ingredient and does not exhibit substantial toxicity with respect to its administration subject. The phrase "not exhibiting substantial toxicity" means that the component is not toxic to an administration subject if it is used at a general dosage. The pharmaceutically acceptable carrier is not particularly limited, and examples thereof include excipients, binders, disintegrants, lubricants, emulsifiers, stabilizers, diluents, solvents for injections, oily bases, moisturizers, touch sensation improvers, surfactants, polymers, thickening/gelling agents, solvents, propellants, antioxidants, reducing agents, oxidizing agents, chelating agents, acids, alkali, powders, inorganic salts, water, metal-containing compounds, unsaturated monomers, polyhydric alcohols, polymer additives, adjuvants, wetting agents, thickeners, tackifiers, oily raw materials, liquid matrices, fat-soluble substances, polymer carboxylate salts, and the like.

Specific examples of these components include those described in PCT International Publication No. WO2016/076310. The pharmaceutically acceptable carrier may be used alone or in combination of two or more kinds thereof.

In addition, the other components are not particularly limited, but examples thereof include preservatives, antibacterial agents, ultraviolet absorbents, whitening agents, vitamins and derivatives thereof, antiphlogistics, anti-inflammatory agents, hair growth agents, blood circulation promoters, stimulants, hormones, anti-wrinkle agents, anti-aging agents, tightening agents, cooling sensation agents, warming sensation agents, wound healing promoters, irritation-relieving agents, analgesics, cell activators, extracts of plants, animals, and microorganisms, antipruritic agents, exfoliating/dissolving agents, antiperspirants, refreshing agents, astringents, enzymes, nucleic acids, fragrances, colorants, coloring agents, dyes, pigments, anti-inflammatory analgesics, antifungals, antihistamines, hypnotic sedatives, tranquilizers, antihypertensives, antihypertensive diuretics, antibiotics, anesthetics, antibacterial substances, antiepileptic agents, coronary vasodilators, herbal medicines, antipruritic agents, keratin softening and exfoliating agents, ultraviolet blockers, antiseptic disinfectants, antioxidant substances, pH adjusters, additives, metal soaps, and the like. Specific examples of these components include those described in PCT International Publication No. WO2016/076310. The other components may be used alone or in combination of two or more kinds thereof.

The composition for promoting glycosaminoglycan production of the present embodiment may be a pharmaceutical composition or a cosmetic preparation.

### (Pharmaceutical composition)

In one embodiment, the present invention provides a pharmaceutical composition for promoting glycosaminoglycan production, containing the above-described glycosaminoglycan-producing promoter, and a pharmaceutically acceptable carrier.

In patients to which the pharmaceutical composition of the present embodiment is administered, the production of glycosaminoglycan in fibroblasts and the like is promoted. As a result, diseases or disorders caused due to glycosaminoglycan deficiency can be prevented or treated. In addition, healing of wounds or burn injuries is promoted.

Accordingly, in one embodiment, the present invention provides a pharmaceutical composition for preventing or treating diseases or disorders caused due to glycosaminoglycan deficiency, the composition containing the above-described glycosaminoglycan-producing promoter, and a pharmaceutically acceptable carrier. In addition, in one embodiment, the present invention provides a pharmaceutical composition for treating wounds or burn injuries, containing the above-described glycosaminoglycan-producing promoter, and a pharmaceutically acceptable carrier.

In the pharmaceutical composition of the present embodiment, the pharmaceutically acceptable carrier is not particularly limited, and carriers generally used for pharmaceuticals can be used in addition to the carriers exemplified above. For example, it is possible to use general raw materials described in the Japanese Pharmacopoeia, the Japanese Pharmaceutical Codex, the Japanese Pharmaceutical Excipients 2013 (Yakuji Nippo, Ltd., 2013), the Japanese Pharmaceutical Excipients Directory 2016 (Edited by International Pharmaceutical Excipients Council Japan, Yakuji Nippo, Ltd., 2016), Handbook of Pharmaceutical Excipients, 7th edition (Pharmaceutical Press, 2012), and the like. The pharmaceutically acceptable carrier may be used alone or in combination of two or more kinds thereof.

In addition to the glycosaminoglycan-producing promoter and the pharmaceutically acceptable carrier, the pharmaceutical composition of the present embodiment may contain other components. The other components are not particularly limited, and general pharmaceutical additives can be used. In addition, active ingredients other than the above-described glycosaminoglycan-producing promoter can be used as the other components. As pharmaceutical additives and active ingredients as the other ingredients, in addition to those exemplified above, it is possible to use general raw materials described in, for example, the Japanese Pharmacopoeia, the Japanese Pharmaceutical Codex, the Japanese Pharmaceutical Excipients 2013 (Yakuji Nippo, Ltd., 2013), the Japanese Pharmaceutical Excipients Directory 2016 (Edited by International Pharmaceutical Excipients Council Japan, Yakuji Nippo, Ltd., 2016), Handbook of Pharmaceutical Excipients, 7th edition (Pharmaceutical Press, 2012), and the like. The other components may be used alone or in combination of two or more kinds thereof.

A dosage form of the pharmaceutical composition of the present embodiment is not particularly limited, and it can be a dosage form generally used for pharmaceutical preparations. Examples thereof include orally administered dosage forms such as tablets, coated tablets, pills, powders, granules, capsules, solutions, suspensions, and emulsions; parenterally administered dosage forms such as injections, suppositories, and external preparations for the skin; and the like. The pharmaceutical composition in these dosage forms can be formulated according to a general method (for example, a method described in the Japanese Pharmacopoeia).

As the pharmaceutical composition of the present embodiment, an external preparation for the skin is preferable. More specific examples of external preparations for skin include dosage forms such as creams, lotions, packs, foams, skin cleansers, extracts, plasters, ointments, spirits, suspensions, tinctures, tapes, poultices, liniments, external aerosols, sprays, and gels.

The pharmaceutical composition of the present embodiment can contain a therapeutically effective amount of the above-described glycosaminoglycan-producing promoter. The term "therapeutically effective amount" refers to an amount of a drug effective for treating or preventing diseases of patients. The therapeutically effective amount may vary depending on a disease state, age, sex, body weight, and the like of an administration subject. In the pharmaceutical composition of the present embodiment, a therapeutically effective amount of the above-described glycosaminoglycan-producing promoter may be an amount at which the inositol derivatives can treat or prevent diseases or disorders caused due to glycosaminoglycan deficiency. In addition, a therapeutically effective amount of the glycosaminoglycan-producing promoter may be an amount at which the inositol derivatives can promote healing of wounds or burn injuries. For example, an therapeutically effective amount of the above-described glycosaminoglycan-producing promoter in the pharmaceutical composition of the present embodiment may be 0.01% to 50% by mass as the content of the inositol derivatives in the pharmaceutical composition, and it may be 0.01 % to 30% by mass for example, it may be 0.01 % to 20% by mass for example, it may be 0.1% to 10% by mass for example, it may be 0.1% to 5% by mass for example, it may be 0.1% to 3% by mass for example, it may be 0.3% to 2% by mass for example, and it may be 0.6% to 1.5% by mass for example.

Regarding the content of the above-described inositol derivatives in the pharmaceutical composition, in a case of using one kind of the inositol derivatives, the content refers to the content of this compound, and in a case of using two or more kinds of the inositol derivatives in combination, the content refers to the total content of these compounds.

A method for administering the pharmaceutical composition of the present embodiment is not particularly limited, and the pharmaceutical composition can be administered by a method generally used as a method for administering pharmaceuticals. For example, it may be administered orally as tablets, coated tablets, pills, powders, granules, capsules, solutions, suspensions, emulsions, and the like; it may be administered intravenously, intraarterially, intramuscularly, intradermally, subcutaneously, intraperitoneally, and the like as an injection, as infusion preparations, and the like alone, or as a mixture with common infusions such as a glucose solution and a Ringer's solution; it may be administered rectally as suppositories; or it may be administered to the skin as external preparations for the skin. In a preferable aspect, the pharmaceutical composition of the present embodiment is applied, affixed, or sprayed to an affected area as an external preparation for the skin.

A dosage of the pharmaceutical composition of the present embodiment can be a therapeutically effective amount. The therapeutically effective amount may be appropriately determined according to symptoms, body weight, age, sex, and the like of a patient, a dosage form of the pharmaceutical composition, an administration method, and the like. For example, in the case of oral administration, a dosage of the pharmaceutical composition of the present embodiment is 0.01 to 500 mg per unit of a dosage form as inositol derivatives; in the case of injections, the dosage is 0.02 to 250 mg per unit of a dosage form as inositol derivatives; in the case of suppositories, the dosage is 0.01 to 500 mg per unit of a dosage form as inositol derivatives; and the like. In the case of external preparations for the skin, for example, the dosage is 0.15 to 500 mg per unit of a dosage form as inositol derivatives, and it may be 0.15 to 300 mg for example, it may be 0.15 to 200 mg for example, and it may be 0.2 to 100 mg for example.

An administration interval of the pharmaceutical composition of the present embodiment may be appropriately determined according to symptoms, body weight, age, sex, and the like of a patient, a dosage form of the pharmaceutical composition, an administration method, and the like. For example, it may be once, about 2 to 3 times, or the like a day.

The pharmaceutical composition of the present embodiment can be administered to, for example, a patient, who has developed a disease or disorder caused due to glycosaminoglycan deficiency, to be used for ameliorating or treating the disease or disorder. In addition, the pharmaceutical composition of the present embodiment can also be used for promoting the production of glycosaminoglycan. Furthermore, the pharmaceutical composition of the present embodiment can also be used for promoting the production of sulfated glycosaminoglycan (particularly N-sulfated glycosaminoglycan) and/or hyaluronic acid. Furthermore, the pharmaceutical composition of the present embodiment can be administered to a patient with a wound or burn injury to be used for ameliorating or treating the wound or burn injury, or for promoting healing of the wound or burn injury. In a preferable aspect, the pharmaceutical composition of the present embodiment is applied, as an external preparation for the skin, to an affected area in which the disease or disorder has developed.

Alternatively, the pharmaceutical composition of the present embodiment can be administered prophylactically to a patient, who is at high risk of developing a disease or disorder caused due to glycosaminoglycan deficiency, to be used for preventing the disease or disorder caused due to glycosaminoglycan deficiency. In a preferable aspect, the pharmaceutical composition of the present embodiment is applied, as an external preparation for the skin, to skin expected to have a high risk of developing the disease or disorder.

In the above description, the diseases or disorders caused due to glycosaminoglycan deficiency are not particularly limited, but examples thereof include decreased skin function (such as residual acne scars), pigmentation, osteoarthrosis, pain disorders (such as arthritis, lower back pain, and nerve pain), and the like. For example, when acne damage reaches the dermis, an amount of glycosaminoglycan produced decreases, causing acne scars to remain in some cases. Since the pharmaceutical composition of the present embodiment promotes the production of glycosaminoglycan, it can be suitably used for treating such acne scars. In this case, the pharmaceutical composition of the present embodiment is preferably applied as an external preparation for the skin to such acne scars.

### (Cosmetic preparation)

In one embodiment, the present invention provides a cosmetic preparation for promoting glycosaminoglycan production, containing the above-described glycosaminoglycan-producing promoter, and a pharmaceutically acceptable carrier.

In skin to which the cosmetic preparation of the present embodiment is applied, the production of glycosaminoglycan in fibroblasts is promoted. As a result, diseases or disorders caused due to glycosaminoglycan deficiency can be ameliorated or inhibited. In addition, healing of wounds or burn injuries is promoted. Accordingly, in one embodiment, the present invention provides a cosmetic preparation for ameliorating or inhibiting a disease or disorder caused due to glycosaminoglycan deficiency, the composition containing the above-described glycosaminoglycan-producing promoter, and a pharmaceutically acceptable carrier. In addition, in one embodiment, the present invention provides a cosmetic preparation for ameliorating a wound or burn injury, or for promoting healing of a wound or burn injury, the cosmetic preparation containing the above-described glycosaminoglycan-producing promoter, and a pharmaceutically acceptable carrier.

In the cosmetic preparation of the present embodiment, the pharmaceutically acceptable carrier is not particularly limited, and carriers generally used for cosmetic preparations can be used in addition to the carriers exemplified above. For example, it is possible to use general raw materials described in the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervised by the Examination Division of the Pharmaceutical Affairs Bureau, Yakuji Nippo, Ltd., 1993); Supplement to the Japanese Cosmetic Ingredients Codex (supervised by the Examination Division of the Pharmaceutical Affairs Bureau, Yakuji Nippo, Ltd., 1993); the Comprehensive Licensing Standards of Cosmetics by Category (supervised by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. The pharmaceutically acceptable carrier may be used alone or in combination of two or more kinds thereof.

In addition to the glycosaminoglycan-producing promoter and the pharmaceutically acceptable carrier, the cosmetic preparation of the present embodiment may contain other components. The other components are not particularly limited, and general additives for cosmetic products can be used. In addition, active ingredients other than the above-described glycosaminoglycan-producing promoter can be used as the other components. As additives for cosmetic products and active ingredients as the other ingredients, in addition to those exemplified above, it is possible to use general raw materials described in, for example, the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervised by the Examination Division of the Pharmaceutical Affairs Bureau, Yakuji Nippo, Ltd., 1993); Supplement to the Japanese Cosmetic Ingredients Codex (supervised by the Examination Division of the Pharmaceutical Affairs Bureau, Yakuji Nippo, Ltd., 1993); the Comprehensive Licensing Standards of Cosmetics by Category (supervised by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. The other components may be used alone or in combination of two or more kinds thereof.

A form of the cosmetic preparation of the present embodiment is not particularly limited, and it can be a form generally used for a cosmetic preparation. Examples thereof include hair cosmetic preparations such as shampoos, hair conditioners, and hairdressing agents; basic cosmetic preparations such as facial cleansers, cleansing agents, skin toners, emulsions, lotions, creams, gels, sunscreens, packs, masks, and serums; makeup cosmetic preparations such as foundations, makeup primers, lipsticks, lip glosses, and blushers; body cosmetic preparations such as body cleansers, body powders, and deodorant cosmetics; and the like. These cosmetic preparations can be manufactured according to a general method. Among them, the cosmetic preparation of the present embodiment is preferably a cosmetic preparation in a form in which it is applied or attached to the skin as an external preparation for the skin. Preferable examples thereof include skin toners, emulsions, lotions, creams, gels, sunscreen agents, packs, masks, serums, foundations, makeup primers, and the like.

A dosage form of the cosmetic preparation of the present embodiment is not particularly limited, but examples thereof include emulsified types such as an oil-in-water (O/W) type, a water-in-oil (W/O) type, a W/O/W type, and an O/W/O type, emulsified polymer types, oily types, solid types, liquid types, kneaded types, stick types, volatile oil types, powder types, jelly types, gel types, paste types, cream types, sheet types, film types, mist types, spray types, multilayer types, foam types, flake types, and the like.

In the cosmetic preparation of the present embodiment, the content of the above-described glycosaminoglycan-producing promoter is not particularly limited, but it can be an effective amount for promoting the production of glycosaminoglycan. For example, a proportion of the above-described glycosaminoglycan-producing promoter in the cosmetic preparation of the present embodiment may be 0.01% to 50% by mass as the content of the inositol derivatives in the cosmetic preparation, and it may be 0.01% to 30% by mass for example, it may be 0.01% to 20% by mass for example, it may be 0.1% to 10% by mass for example, it may be 0.1% to 5% by mass for example, it may be 0.1% to 3% by mass for example, it may be 0.3% to 2% by mass for example, and it may be 0.6% to 1.5% by mass for example.

Regarding the content of the above-described inositol derivatives in the cosmetic preparation, in a case of using one kind of the inositol derivatives, the content refers to the content of this compound, and in a case of using two or more kinds of the inositol derivatives in combination, the content refers to the total content of these compounds.

An amount of the cosmetic preparations used of the present embodiment is not particularly limited, but it can be an effective amount for promoting the production of glycosaminoglycan. For example, an amount of the cosmetic preparations used of the present embodiment may be 0.15 to 500 mg per use as an amount of inositol derivatives, and it may be 0.15 to 300 mg for example, it may be 0.15 to 200 mg for example, and it may be 0.2 to 100 mg for example.

A use interval of the cosmetic preparation of the present embodiment is not particularly limited, but it can be, for example, once, about 2 to 3 times, or the like a day.

The cosmetic preparation of the present embodiment can be applied to, for example, a site (such as the face, limbs, elbow joints, and knee joints), which has a high risk of developing a disease or disorder caused due to glycosaminoglycan deficiency, to be used for inhibiting the onset of the disease or disorder caused due to glycosaminoglycan deficiency. In addition, in a case where symptoms such as decreased skin function (such as residual acne scars) and pigmentation appear, amelioration and prevention of the symptoms, or inhibition of progression of the symptoms can be expected when using the cosmetic preparation of the present embodiment for daily skin care and makeup. Furthermore, promotion of healing of a wound or burn injury can be expected when using the cosmetic preparation of the present embodiment for skin care of wounded or burn-injured skin.

### [Other Embodiments]

In one embodiment, the present invention provides a method for promoting the production of glycosaminoglycan, including a step of administering, to a mammal, an inositol derivative in which a sugar (a monosaccharide or an oligosaccharide) binds to inositol.

In one embodiment, the present invention provides a method for promoting the production of sulfated glycosaminoglycan (particularly N-sulfated glycosaminoglycan), including a step of administering, to a mammal, an inositol derivative in which a sugar (a monosaccharide or an oligosaccharide) binds to inositol.

In one embodiment, the present invention provides a method for promoting the production of hyaluronic acid, including a step of administering, to a mammal, an inositol derivative in which a sugar (a monosaccharide or an oligosaccharide) binds to inositol.

In one embodiment, the present invention provides a method for preventing or treating a disease or disorder (decreased skin function (such as residual acne scars), pigmentation, osteoarthrosis, pain disorders (such as arthritis, lower back pain, and nerve pain), and the like) which is caused due to glycosaminoglycan deficiency, the method including a step of administering, to a mammal, an inositol derivative in which a sugar (a monosaccharide or an oligosaccharide) binds to inositol.

In one embodiment, the present invention provides a method for promoting the treatment of a wound or burn injury, including a step of administering, to a mammal, an inositol derivative in which a sugar (a monosaccharide or an oligosaccharide) binds to inositol.

In one embodiment, the present invention provides an inositol derivative, in which a sugar (a monosaccharide or an oligosaccharide) binds to inositol, for use in promoting the production of glycosaminoglycan.

In one embodiment, the present invention provides an inositol derivative, in which a sugar (a monosaccharide or an oligosaccharide) binds to inositol, for use in promoting the production of sulfated glycosaminoglycan (particularly N-sulfated glycosaminoglycan).

In one embodiment, the present invention provides an inositol derivative, in which a sugar (a monosaccharide or an oligosaccharide) binds to inositol, for use in promoting the production of hyaluronic acid.

In one embodiment, the present invention provides an inositol derivative, in which a sugar (a monosaccharide or an oligosaccharide) binds to inositol, for preventing or treating a disease or disorder (decreased skin function (such as residual acne scars), pigmentation, osteoarthrosis, pain disorders (such as arthritis, lower back pain, and nerve pain), and the like) which is caused due to glycosaminoglycan deficiency.

In one embodiment, the present invention provides an inositol derivative, in which a sugar (a monosaccharide or an oligosaccharide) binds to inositol, for promoting healing of wounds or burn injuries.

In one embodiment, the present invention provides use of an inositol derivative, in which a sugar (a monosaccharide or an oligosaccharide) binds to inositol, for the manufacture of a glycosaminoglycan-producing promoter.

In one embodiment, the present invention provides use of an inositol derivative, in which a sugar (a monosaccharide or an oligosaccharide) binds to inositol, for the manufacture of a sulfated glycosaminoglycan-producing promoter (particularly an N-sulfated glycosaminoglycan-producing promoter).

In one embodiment, the present invention provides use of an inositol derivative, in which a sugar (a monosaccharide or an oligosaccharide) binds to inositol, for the manufacture of a hyaluronic acid-producing promoter.

In one embodiment, the present invention provides use of an inositol derivative, in which a sugar (a monosaccharide or an oligosaccharide) binds to inositol, for the manufacture of a composition for promoting glycosaminoglycan production.

In one embodiment, the present invention provides use of an inositol derivative, in which a sugar (a monosaccharide or an oligosaccharide) binds to inositol, for the manufacture of a composition for promoting sulfated glycosaminoglycan production (particularly a composition for promoting N-sulfated glycosaminoglycan production).

In one embodiment, the present invention provides use of an inositol derivative, in which a sugar (a monosaccharide or an oligosaccharide) binds to inositol, for the manufacture of a composition for promoting hyaluronic acid production.

In one embodiment, the present invention provides use of an inositol derivative, in which a sugar (a monosaccharide or an oligosaccharide) binds to inositol, for the manufacture of a composition for preventing or treating diseases or disorders (decreased skin function (such as residual acne scars), pigmentation, osteoarthrosis, pain disorders (such as arthritis, lower back pain, and nerve pain), and the like) which are caused due to glycosaminoglycan deficiency.

In one embodiment, the present invention provides use of an inositol derivative, in which a sugar (a monosaccharide or an oligosaccharide) binds to inositol, for the manufacture of a composition for promoting healing of wounds or burn injuries.

### [Examples]

Hereinafter, while the present invention will be described with reference to the following experimental examples, the present invention is not limited to the following experimental examples.

### [Experimental Example 1]

### (Production of inositol derivatives)

Myo-inositol and β-cyclodextrin were reacted in the presence of a cyclodextrin glucanotransferase to produce inositol derivatives in which glucose, or an oligosaccharide having glucose as a monosaccharide unit bound to the myo-inositol. As a result of analyzing the produced inositol derivatives by liquid chromatography-mass spectrometry (LC-MS), a percentage of molecules in which the number of glucose molecules in a glucose chain which bound to the myo-inositol was one was 12% by mass, a percentage of molecules in which the above number of glucose molecules was two was 30% by mass, a percentage of molecules in which the above number of glucose molecules was three was 9% by mass, a percentage of molecules in which the above number of glucose molecules was four was 12% by mass, and a percentage of molecules in which the above number of glucose molecules was five was 2% by mass.

### [Experimental Example 2]

### (Medium preparation and cell culture)

As cells, cells obtained by treating normal human fibroblasts (RIKEN BioResource Research Center) with hydrogen peroxide were used. In addition, as a medium, Dulbecco's Modified Eagle's Medium (DMEM) containing 10% fetal bovine serum was used.

The inositol derivatives produced in Experimental Example 1 were dissolved in purified water and added to the medium so that a final concentration became 10⁻⁴ w/v%, and thereby a medium was prepared (Example 1). Furthermore, as a comparative example, a medium in which myo-inositol was added instead of the inositol derivatives was prepared (Comparative Example 1).

Furthermore, as a control, a medium in which purified water was added instead of the inositol derivatives was prepared (control example). The cells were cultured in these media for 72 hours at 37°C in a 5% CO₂ atmosphere.

### [Experimental Example 3]

### (Effect of promoting glycosaminoglycan production)

Proteoglycan present in dermal matrices is a glycoprotein that consists of a sugar chain part called a glycosaminoglycan and a core protein. Examples of glycosaminoglycans include hyaluronic acid, heparan sulfate (including heparin), keratan sulfate, dermatan sulfate, and the like. The present experimental example shows the influence of the inositol derivatives on glycosaminoglycan production in normal human fibroblasts.

Cells were cultured as described in Experimental Example 2. After 72 hours from the start of culture, the medium was removed, and the cells were washed with phosphate-buffered saline. Thereafter, the cells were treated with a buffer solution containing papain, and the cells were recovered. Using this as a sample, a total amount of sulfated glycosaminoglycan was measured using Blyscan Dye (1,9-dimethyl-methylene blue) that specifically binds to sulfated sugar chains by a colorimetric method.

A nitrous acid solution was added to the same sample to decompose N-sulfated glycosaminoglycan in the sample, and then O-sulfated glycosaminoglycan was quantitatively determined. An amount of N-sulfated glycosaminoglycan was obtained by subtracting an amount of O-sulfated glycosaminoglycan from the total amount of sulfated glycosaminoglycan.

For quantification of a total amount of sulfated glycosaminoglycan and O-sulfated glycosaminoglycan, a glycosaminoglycan quantification kit (Blyscan Glycosaminoglycan Assay Kit, Funakoshi) was used.

The results are shown in Table 1. Comparing to the purified water (control example), both a total amount of sulfated glycosaminoglycan and an amount of N-sulfated glycosaminoglycan increased in the group to which the inositol derivatives were added (Example 1). Meanwhile, comparing to the purified water (control example), no significant change in the total amount of sulfated glycosaminoglycan and the amount of N-sulfated glycosaminoglycan was recognized in the group to which myo-inositol was added (Comparative Example 1).

**[Table 1]**

| | Drug | Concentration after addition (w/v%) | Total sulfated glycosaminoglycan | N-Sulfated glycosaminoglycan |
|---|---|---|---|---|
| | | | Chondroitin sulfate, keratan sulfate, dermatan sulfate, heparan sulfate, heparin | Heparan sulfate, heparin |
| Control example | Purified water | - | 5.42 ± 0.65 | 3.10 ± 0.57 |
| Comparative Example 1 | Myo-inositol | 10⁻⁴ | 5.10 ± 0.31 | 2.93 ± 0.29 |
| Example 1 | Inositol derivative | 10⁻⁴ | 7.10 ± 0.44 | 4.75 ± 0.44 |

| | | | | |
|---|---|---|---|---|
| Unit: µg/well | | | | |

### [Experimental Example 4]

### (Effect of promoting hyaluronic acid production)

The present experimental example shows the influence of the inositol derivatives on hyaluronic acid production in normal human fibroblasts.

Cells were cultured as described in Experimental Example 2. After 72 hours from the start of culture, a medium was recovered, and the cells were washed with phosphate-buffered saline. Thereafter, a cell survival rate was determined by a neutral red method.

An amount of hyaluronic acid in the recovered medium was measured using a hyaluronic acid measurement kit (Iwai Chemicals Company, PG Research Inc.) by a competition method in which a biotin-labeled hyaluronic acid-binding protein is used.

The results are shown in Table 2. Comparing to the purified water (control example), the amount of hyaluronic acid increased in the group to which the inositol derivatives were added (Example 1). Meanwhile, comparing to the purified water (control example), no significant change in the amount of hyaluronic acid was recognized in the group to which myo-inositol was added (Comparative Example 1).

**[Table 2]**

| | Drug | Concentration after addition (w/v%) | Hyaluronic acid |
|---|---|---|---|
| Control example | Purified water | - | 53.3 ± 7.3 |
| Comparative Example 1 | Myo-inositol | 10⁻⁴ | 59.0 ± 12.5 |
| Example 1 | Inositol derivative | 10⁻⁴ | 86.8 ± 9.1 |

| | | | |
|---|---|---|---|
| Unit: ng/mL-medium | | | |

Based on the above results, it was clarified that the inositol derivatives act to promote glycosaminoglycan production in human fibroblasts.

### [Industrial Applicability]

According to the present invention, it is possible to provide a glycosaminoglycan-producing promoter and a composition for promoting glycosaminoglycan production which can promote the production of both a sulfated glycosaminoglycan and hyaluronic acid.

## Claims

1. A glycosaminoglycan-producing promoter comprising, as an active ingredient, an inositol derivative in which a sugar binds to inositol.

2. The glycosaminoglycan-producing promoter according to Claim 1, wherein the sugar is a monosaccharide or an oligosaccharide.

3. The glycosaminoglycan-producing promoter according to Claim 2, wherein the monosaccharide is glucose.

4. The glycosaminoglycan-producing promoter according to Claim 2, wherein the oligosaccharide is an oligosaccharide containing glucose as a structural unit.

5. The glycosaminoglycan-producing promoter according to any one of Claims 1 to 4, wherein the inositol is myo-inositol.

6. The glycosaminoglycan-producing promoter according to any one of Claims 1 to 5, wherein the glycosaminoglycan is a sulfated glycosaminoglycan.

7. The glycosaminoglycan-producing promoter according to any one of Claims 1 to 5, wherein the glycosaminoglycan is hyaluronic acid.

8. A composition for promoting glycosaminoglycan production, comprising:
the glycosaminoglycan-producing promoter according to any one of Claims 1 to 7; and
a pharmaceutically acceptable carrier.

9. The composition for promoting glycosaminoglycan production according to Claim 8, wherein the content of the inositol derivative is 0.01% to 50% by mass.

10. The composition for promoting glycosaminoglycan production according to Claim 8 or 9, which is an external preparation for skin.

11. The composition for promoting glycosaminoglycan production according to any one of Claims 8 to 10, which is a cosmetic preparation.
